# EUROPEAN PATENT APPLICATION

(11) **EP 2 743 343 A1**
(43) Date of publication of application: **18.06.2014**
(21) Application number: 12822834.3
(22) Date of filing: 07.08.2012
(51) Int. Cl.: C12N 5/0775, A61K 9/12, A61K 35/36, A61P 17/02

(54) **SPRAY FORMULATION OF MESENCHYMAL STEM CELLS FOR THE TREATMENT OF CHRONIC WOUNDS**

(30) Priority: 08.08.2011 CL 20111904
(71) Applicant: Pontificia Universidad Católica de Chile, Santiago (CL)
(72) Inventor: EBENSPERGER, Roberto, Santiago (CL)
(74) Representative: Schiweck, Weinzierl & Koch
(86) International application number: PCT/CL2012/000041
(87) International publication number: WO 2013/020242

(57) **Abstract**

The present Invention describes therapeutic and medical applications of Mesenchymal Stem Cells, by means of fine mist spraying application system. The mesenchymal stem cells used in the fine mist spray application system are in suspension and may optionally be nanoencapsulated or not.

## Description

The present Invention describes therapeutic and medical applications related to cell-based therapy using Mesenchymal Stem Cells.

The therapeutic applications described in the present Invention are related to a fine mist spray application system of mesenchymal stem cells derived from human adipose tissue for topical application on wounds and burns in general. Said application may be extended to be used in animals, obtaining stem cells of animal origin.

The mesenchymal stem cells may be incorporated into the spray application system in the form of a suspension of uncoated intact cells, as well as nano-encapsulated stem cells.

### STATE OF THE ART

Cell-based therapy seeks to solve physiological and physio-pathological deficiencies by means of the administration and/or implantation of live cells and/or tissues into an organism. These cells and/or tissues are classified according to their source, there being autologous (from the very same patient), allogeneic (from other human donors) and xenogeneic (from other animal species) implants. In ideal conditions, the most recommended source for these therapies is from the patients themselves (autologous), because in this case one avoids implant rejection in the organism, as it does not recognize the implant as a foreign agent (1).

Allogeneic and xenogeneic sources have a potential use in the pharmaceutical and clinical markets, given that it is more feasible to obtain, culture and standardize all processes, before reaching the patient. However, xenogeneic implants have a lower biosafety profile and for this reason, the use of allogeneic implants is preferred. The appropriate conditions and technologies are currently being developed so that these treatments reach as many patients as possible (1).

Regarding allogeneic and xenogeneic implants, the cell-based therapy shows some limitations, mainly in the decrease in cell viability due to the inflammatory environment of the implantation site and the generation of a possible immune response, which may lead to the rejection of the transplanted cells and tissues and their subsequent destruction (2).

Furthermore, there are limitations about the inherent fragility of mammalian cells, as a result of cell stress to which they are subjected before being administered to a patient, including cell handling, transport and storage. In addition to these adverse conditions, one must consider the metabolic requirements of cells that are necessary to ensure cell viability (2, 3).

In order to overcome these limitations, multidisciplinary research has used tools inspired by tissue engineering, biotechnology and nanotechnology. When applied to biomedicine, this science is encompassed under the concept that researchers are calling *Bionanotechnology.*

Stem Cells (SC).

The utilization of stem cells represents one of the most promising alternatives for cell-based therapy and tissue engineering, given that they have the potentiality to differentiate themselves into multiple cell types and to secrete growth factors that stimulate tissue growth and regeneration (4). What is particularly novel is the use of mesenchymal stem cells of allogeneic origin, thanks to their low potential of immunogenicity and their immunomodulatory capacity (5, 6).

There are different types of stem cells; they are classified according to the capacities of proliferation, differentiation, of the extraction tissue and of the embryonic germ lines thereof.
- Totipotent Stem Cells: cells having the potential to give rise to the totality of an organism's tissues and extraembryonic tissues, and that can replicate themself, too. In this case, there is only one cell type with these characteristics, namely the Embryonic Stem Cell (ESC), which originates immediately after fertilization.
- Pluripotent Stem Cells: cells formed in the three embryonic layers - the endoderm, the mesoderm and the ectoderm. Said cells give rise to the various types of tissues corresponding to their own cell line, and maintain the capacity of self-regeneration.
- Multipotent Stem Cells: cells of adult origin, present in small proportions in each tissue of an organism and possessing a certain level of differentiation, given that they come from the different germ lines. These cells are able to self-regenerate and to differentiate themselves both *in-vitro* and *in-vivo* to the respective cellular subtype of the tissue of origin.

Most stem cell studies, including human clinical trials, have used Hematopoietic Stem Cells (HSC) and also Mesenchymal Stem Cells (MSC). Both multipotent cells originate in the bone marrow (7).

The MSC can also be obtained from human Adipose-Derived tissue Stem Cells (ADSC) (8). Unlike other sources, these stem cells allow for a less invasive form of obtaining, with no transgressions or moral conflicts, because the adipose tissue from which they are obtained (such as liposuctions and abdominoplasties) is normally regarded as waste.

Being multipotent cells, the aforementioned MSC are able to give rise by cell differentiation, in *in-vitro* conditions, to different cellular subtypes including adipocytes, osteocytes, chondrocytes, myocytes, skin, neurons, hematopoietic cells and other types of connective tissue (7).

The stem cells used in the present Invention specifically correspond to ADSC in suspension incorporated to the atomization (spray) formulation, whether in nanoencapsulated or non encapsulated form.

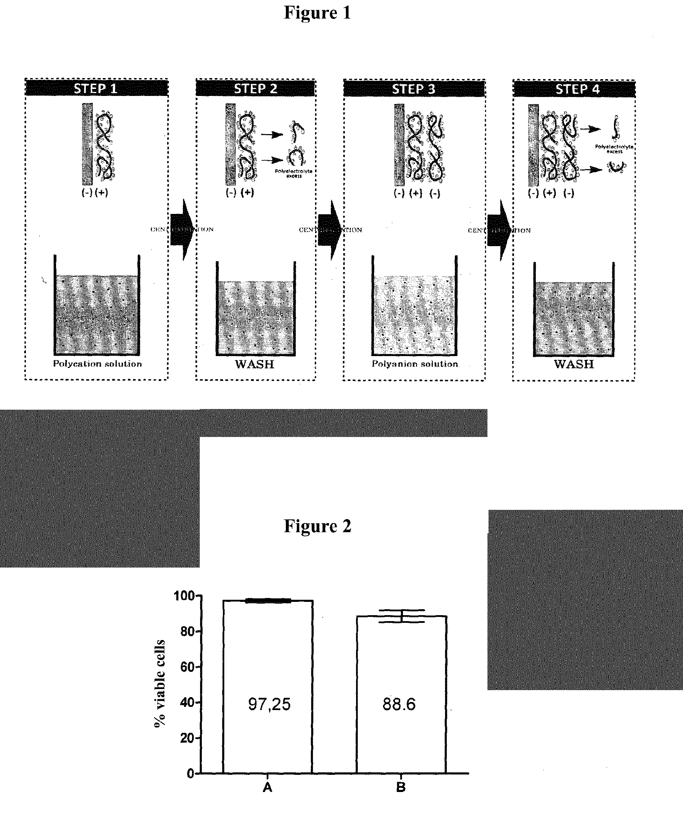

Nanoencapsulation basically arises from a technique known as layer-by-layer deposition, a process initially set forth by Langmuir comprising the alternating deposition of fine layers of polyelectrolytes (having nanometric thickness) with opposite surface charges on a charged template; it is therefore a self-assembly process brought about by electrostatic interactions (Figure 1). This method is used in a wide range of useful applications for the industry, science and technology, given that it is a simple, low-cost method (9).

The characteristics of the nanocapsules created with the layer-by-layer method may be manipulated by factors such as; the type of polyelectrolyte, concentration, molecular mass, number of layers, charge of the final layer, pH, salts present, and incubation time in the solution where the deposition/sedimentation takes place. Said factors modify characteristics such as pore size, surface charge density, elasticity, and degree of hydration of the nanocapsules, determining the permeability, release kinetics, and mechanical resistance, among other, of the nanocapsules (10, 11).

The ADSC are used as a template (having a negative net charge), wherein nanoencapsulation is a carrier that enables the application of these cells while overcoming some limitations associated to cell-based therapy and to other encapsulation types used (12).

The currently known applications of stem cells for the treatment of wounds mainly comprise the systemic infusion into vascular circulation, and the direct application on the site of the wound (13).

The direct applications onto a wound site also include a description of the topical application of an autologous culture of mesenchymal stem cells (MSC) derived from bone marrow, to accelerate the healing of skin wounds. This system comprises a dual-chamber syringe: in the first chamber, stem cells containing a fibrinogen solution are resuspended; while the second chamber of the syringe contains a diluted thrombin solution. The MSC delivery system has been described as a spray with a syringe (14). Said system does not really correspond to a spray, because it does not involve atomization of the suspension applied directly to wounds.

The low long-term survival of the cells in the formulation constitutes a disadvantage of products formulated on the basis of live cells, regardless of the chosen means of administration. Cell viability decreases over time under any storage condition, whether it is at 4°C or at room temperature. Therefore, this problem is common to any pharmaceutical preparation that utilizes live cells. For this reason, in the prior art there is no live cell formulation for application by means of atomization (fine mist spraying), as considerable cell damage would be expected after an atomized application.

The state of the art does not describe stem cell applications via spray, as is described in the present Invention. Some related application systems are known, such as the product Teseel®, corresponding to a surgical sealant based on fibrin that does not incorporate stem cells.

The company Healthpoint Ltd. Has developed a wound therapy based on experimental cellular therapy for the treatment of venous leg ulcers. The product consists of two components that are administered topically and sequentially on the wound: a fibrinogen solution and a suspension of live allogeneic dermal fibroblasts and epidermal keratinocytes. The product would release a series of growth and angiogenic factors into the microenvironment at the wound bed, a result of the metabolic activity of these cells that are administered alive and that are captured on the surface of the wound by means of a fibrin mesh. The aforementioned product does not comprise any type of stem cells either, nor is the form of application by means of a fine mist spray.

An important aspect to consider has to do with cell viability following an atomization process. The passage of the cells through the atomizer valve could damage the cells, causing their rupture due to physical effects. However, it has been demonstrated that this phenomenon is minimal because the cell viability is maintained (Figure 2). In other words, although there is a tendency to recovering fewer live cells after spraying, the difference does not reach statistical significance. The Inventors have found an unexpected and surprising result upon determining that there is no significant damage to the stem cells when subjecting them to the spraying process, with the evident advantages of applying said stem cells in a new application form and preserving the cell properties intact.

Up to date there is no knowledge of stem cell spray applications for the treatment of wounds, and the most commonly used systems do not offer the advantages described in the present Invention. In the case of advanced healing dressings, we can mention as a disadvantage, that none of the dressings currently in use possesses by itself all the ideal requirements or characteristics that a dressing should present to treat all wound types (necrotic, sloughing, granulating and epithelializing wound).

The provision of a single effective dressing or treatment for all chronic wounds is not commercially available nor has it been published in the prior art.

Besides the difficulty of the nonexistence of a single dressing system for different wound types, the healing of acute wounds is only partially effective, given that 50% of patients do not achieve the healing of their wounds with this therapy, a situation that is even more complex if the wound is over a year old (15).

This further entails that if the wounds heal, they take longer to cicatrize and, depending on the seriousness of the wound, it is necessary to continuously evaluate what kind of dressing to use and when to use each dressing as the cicatrization or healing advances (16).

Due to the lack of high quality research results in connection with the effectiveness of the various existing supports or dressings, it can be said that the application of treatments - and therefore the selection of the correct dressing in the daily clinical practice for handling wounds - is based more on the knowledge and/or experience and/or costs and/or personal preference of the doctor as to the dressing, rather than on purely scientific evidence.

The present Invention shows advantages that conventional treatments for healing wounds do not. Among the advantages of the fine mist spray application system of the present Invention, we can mention the following:
- The fine mist spray application allows an easy application due to the simplicity of the system used (atomizer). The presence of a qualified or trained person is not required for applying the product on the wound. It can be applied by the patients themselves or by a third party, without any specialized instruction.
- The fine mist spray application allows an even distribution, determining that a uniform film or layer is formed or deposited on the entire affected area.
- The fine mist spray application allows an easy use due to less manipulation for its formulation. The reason for this is that the application system of the present Invention does not require the presence of fibrin and thrombin and therefore it does not coagulate.
- The fine mist spray application causes less pain than other treatments. Less manipulation of the wound entails less pain for the patient during application of the product, which in practice means more patient acceptance, satisfaction and comfort.
- The fine mist spray application makes it useful on any wound type; that is; necrotic, sloughing, granulating and epithelializing.
- The fine mist spray application allows it to be useful for any patient in need thereof, whether it is a newborn, a baby, a child, an adult, or an elderly.
- The fine mist spray application allows shorter nursing care times.
- The fine mist spray application makes it possible for treatment to be performed on an out-patient basis, reducing the hospitalization periods and improving the patients' quality of life, which contributes to improvements in the patients' disposition and their healing results.
- In addition to the previously described advantages offered by an administration via atomization or "fine mist spray", the nanoencapsulated cells formulated according to the Invention present another surprising characteristic: an enhanced resistance to physical stress (mechanical resistance) and therefore a minimization of the cell damage suffered during the atomization process, that is, during the passage of the cells through the atomizer. This can be determined because the nanoencapsulated cell viability after atomization is greater than that of non nanoencapsulated cells (Figure 3); moreover their functionality, that is, their proliferation capacity (Figure 4) and differentiation capacity are not altered (Figure 5).

### DESCRIPTION OF THE FIGURES

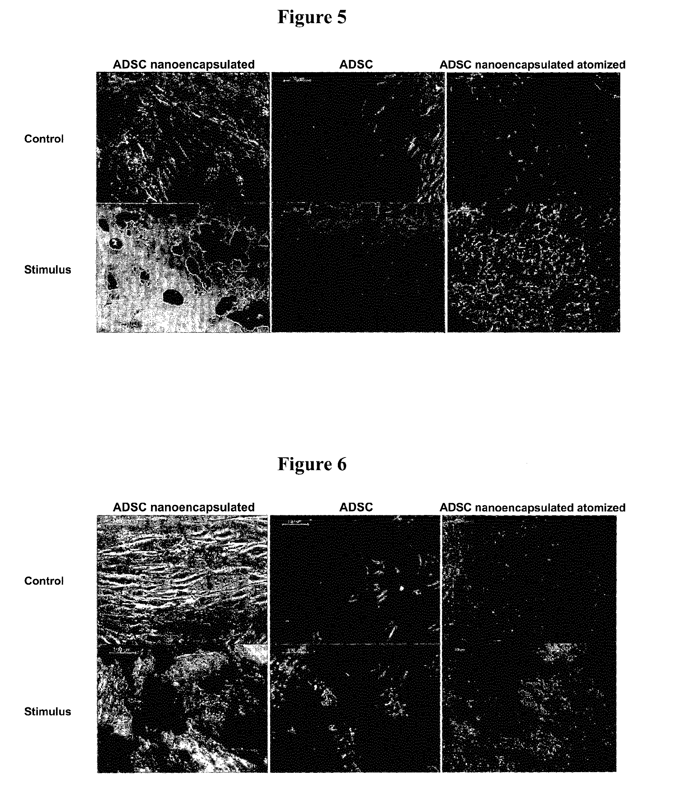

Figure 1: Layer-by-layer deposition cycle on a charged template. Stage 1: polycation solution; Stage 2: wash; Stage 3: polyanion solution; Stage 4: wash.
Figure 2: Cell viability after fine mist spraying. The Figure shows the percentage of cell viability of mesenchymal stem cells derived from human adipose tissue before (A) and after (B) being atomized (sprayed). The y axis corresponds to the percentage of viable cells, it being 97.25% before the fine mist spraying, and 88.6% after the fine mist spraying. The values did not reach a significant difference (n=3).

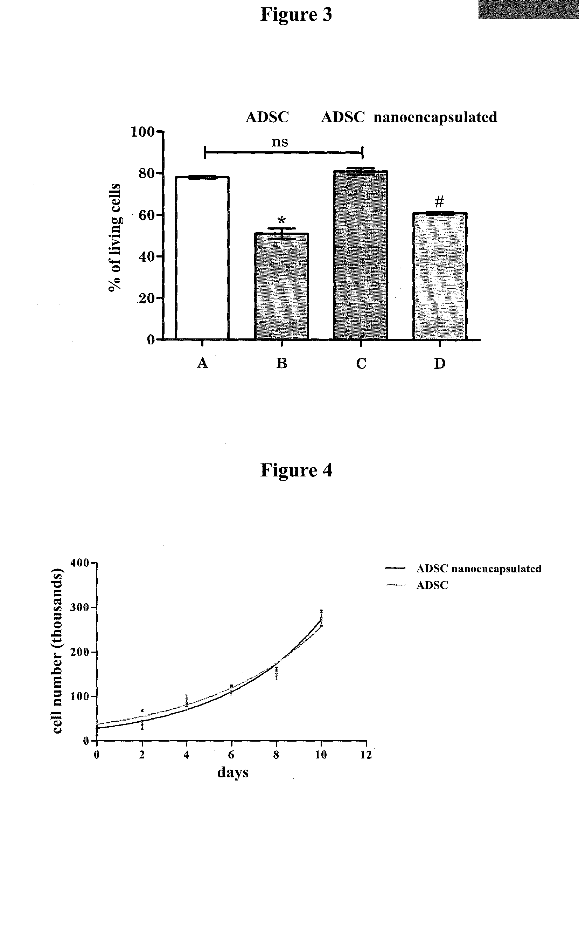

Figure 3: Cell viability of nanoencapsulated and non nanoencapsulated cells after fine mist spraying. The Figure indicates the percentage of cell viability of MSC derived from human adipose tissue, both nanoencapsulated and non-nanoencapsulated, before and after fine mist spraying (atomization). A: Pre-atomization non nanoencapsulated cells; B: Post-atomization non nanoencapsulated cells; C: Pre-atomization nanoencapsulated cells; D: Post-atomization nanoencapsulated cells. It is observed that that nanoencapsulation process (Bar C) does not affect cell viability with regard to non nanoencapsulated cells (Bar A) (NS). However, after the cells are fine-mist sprayed, it can be seen that the nanoencapsulated cells resist the atomization process better, a difference that does reach statistical significance (p<0.05). (*) and (#) present a value of p<0.05 with regard to the pre-atomization control thereof. (n=3; NS: not significant).
Figure 4: Cell proliferation of nanoencapsulated and non nanoencapsulated mesenchymal ADSC. The Figure shows that the proliferation curves of the nanoencapsulated and non nanoencapsulated mesenchymal stem cells do not present differences.
   Nanoencapsulated ADSC: y=28.3 e^{0.227x}, k=0.227, Tau=4.41, Doubling time = 3.06, r² = 0.963
   Non nanoencapsulated ADSC: y=37.6 e^{0.192x}, k=0.192, Tau=5.20, Doubling time = 3.60, r² = 0.929
Figure 5: Differentiation of nanoencapsulated and non nanoencapsulated mesenchymal ADSC to adipocytes before and after atomization. The photographs show images obtained under microscope (200x) of the non nanoencapsulated as nanoencapsulated ADSC, before and after atomization, and subjected to differentiation stimulus to adipocyte, which is made evident by staining with Oil red O. The images show that the nanoencapsulation process as well as the atomization process does not affect the differentiation capacity of the ADSC in adipocytes.
Figure 6: Differentiation of nanoencapsulated and non nanoencapsulated mesenchymal ADSC to osteoblasts before and after atomization. The photographs show images obtained under microscope (200x) of the non nanoencapsulated as nanoencapsulated ADSC, before and after atomization, and subjected to differentiation stimulus to osteoblasts, which is made evident by staining with Alizarin red. The images show that the nanoencapsulation process as well as the atomization (fine mist spraying) process does not affect the differentiation capacity of the ADSC in osteoblasts.

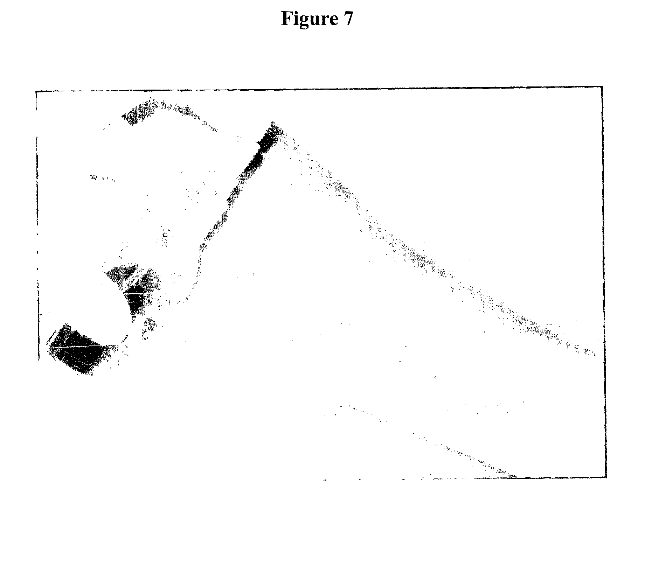

Figure 7: Live cell application system by means of fine mist spray system. The image shows the live cell application system based on fine mist spraying, which comprises a container or vessel having an atomization system and the pharmaceutical formulation wherein the active ingredient consists in live cells suspended in a pharmaceutically acceptable carrier.

### SPECIFICATIONS OF THE INVENTION

The present Invention describes a fine-mist spray application system of a formulation comprising, as active ingredient, nanoencapsulated and non nanoencapsulated mesenchymal stem cells, suspended in a carrier, for topical application on wounds and burns in humans and animals. To this end, mesenchymal stem cells will be used, incorporated as a suspension in appropriate carriers to be applied by means of a fine mist atomizer (of the "GMSP Pre-compressed Fine Mist Spray Pump" type).

One of the objectives of the present Invention is to formulate a stem cell suspension in an adequate carrier to be applied by atomization (fine mist spray), wherein said cells present the necessary mechanical resistance to maintain their viability and functionality after the atomization process.

Another objective of the present Invention is to formulate a suspension of mesenchymal stem cells derived from human adipose tissue, ADSC (usually lipo-aspirated) whose minimum phenotype is: positive to CD105, CD90, CD73 and negative for CD14, CD19, CD45 and HLA-DR, to be incorporated in the fine mist spray system.

A further objective of the present Invention is to formulate a suspension of nanoencapsulated mesenchymal stem cells derived from human adipose tissue, ADSC (usually lipo-aspirated) whose minimum phenotype is: positive to CD105, CD90, CD73 and negative for CD14, CD19, CD45 and HLA-DR, to be incorporated in the fine mist spray system.

The used mesenchymal stem cells show the capacity of differentiating themselves into adipose cells, osteoblasts and chondrocytes.

The carrier used for the stem cell formulation consists in a pharmaceutically acceptable carrier for suspending the cells, ensuring the viability and integrity of the cells during a set period of time that comprises between 1 hour and 72 hours (3 days).

The pharmaceutically acceptable carriers used in the formulation of the present Invention are selected from amongst a group that includes; saline solution, phosphate buffered saline (PBS), Ringer's serum, Ringer's lactate serum, autologous serum, and AB donor serum.

Other possible excipients of the formulation correspond to; 4-(2-hydroxyetyl)-1-piperazine-ethanosulfonic acid (HEPES) buffer, bicarbonate buffer, FDC coloring agents (for example, Blue N°1 and Blue N°2), heparins, platelet lysates, and N-acetyl cysteine (NAC).

The utilized carrier may contain agents that promote the wound healing processes, and antibiotic substances to ensure the sterility of the product.

The agents that promote the wound healing processes may be selected from among the group that includes; chitosan, hyaluronic acid, allantoin, type 1 collagen, a decoction or extract (tannins) of matico (*Piper angustifolium*)*,* a tincture of hydrocotyle or *Centella asiatica* (could be alcoholic), decoctions of calendula *(Calendula officinalis)* and Aloe Vera extracts.

The agents that ensure product sterility can be selected from among the group that includes: penicillin, streptomycin, gentamicin, quinolones (for example, Ciprofloxacin), silver sulfadiazine, chlorhexidine, quaternary ammonium derivatives (for example, Benzalkonium chloride) and lactoferrin.

Once formulated, the stem cell suspension is incorporated into a container or vessel made of sterile glass having a threaded neck so as to allow adjusting a fine mist spray system. The type of atomizer used is of the GMSP Pre-compressed Fine Mist Spray Pump type.

The volume of the vessel may range between 5 mL and 20 mL depending on the surface of the wound that must be covered (considering surfaces from about 5 to about 300 cm²).

Cell concentrations inside the spray may vary between 5 x 10⁵ and 4 x 10⁶ cells per mL.

### EXAMPLES

The preferred embodiments of the present Invention are described with the help of the following examples: said examples do not constitute a limitation of the present Invention.

### Example 1:

### Formulation of SC in Suspension:

The stem cells were obtained by following established protocols previously described in the state of the art (17, 18).

The ADSC are separated from lipoaspirate or abdominal adipose tissue using collagenase. After the digestion of said enzyme, the adipocytes are separated from the stromal vascular fraction (SVF) by centrifugation. The erythrocytes are lysed and the stem-cell enriched SVF is washed in PBS and filtered through a 100 µm mesh sieve. The cells are centrifuged and seeded in conventional culture bottles in DMEM + 10% FBS and antibiotics. Cell adherence is allowed during a period of between 24 and 48 hours. At the end of this period, the culture medium is substituted with fresh medium. The cells are fed every 3 days and cultured at 37°C in a moist atmosphere of 5 % CO₂.

### Example 2:

### Formulation of Nanoencapsulated SC in Suspension:

The stem cells were obtained by following established protocols previously described in the prior art (17, 18).

The ADSC are separated from lipoaspirate or abdominal adipose tissue using collagenase. After the digestion of said enzyme, the adipocytes are separated from the stromal vascular fraction (SVF) by centrifugation. The erythrocytes are lysed and the stem-cell enriched SVF is washed in PBS and filtered through a 100 µm mesh sieve. The cells are centrifuged and seeded in conventional culture bottles in DMEM + 10% FBS and antibiotics. Cell adherence is allowed during a period of between 24 and 48 hours. At the end of this period, the culture medium is substituted with fresh medium. The cells are fed every 3 days and cultured at 37°C in a moist atmosphere of 5 %CO₂. The cells obtained in this manner are subjected to the nanoencapsulation process in keeping with the following protocol.

### Nanoencapsulation Protocol:

1. Obtain a cell suspension from a culture plate or bottle.
2. Carry out 2 washes with *Hank's Balanced Salt Solution* (HBSS) to eliminate the presence of culture medium, using centrifugation at 200 g during 5 minutes; remove supernatant and resuspend cell pellet in HBSS.
Preparation for 1 Liter of HBSS (make up to final volume by using Milli Q grade water):

| Components | Concentration (mM) | Mass (mg) |
|---|---|---|
| CaCl₂(dihydrate) | 1.3 | 186 |
| MgSO₄ (heptahydrate) | 0.814 | 200 |
| NaCl | 137 | 8000 |
| KCI | 5.4 | 400 |
| KH₂PO₄ | 0.44 | 60 |
| Na₂HPO₄ | 0.338 | 48 |
| NaHCO₃ | 4.2 | 350 |
| Glucose | 5.55 | 1000 |

3. Resuspend the pellet from the second wash in a polycation solution (Chitosan 500 µg/mL, low molecular weight 448869 Sigma-Aldrich), considering a volume ratio between pellet and polycation of 1:10. Incubate during 10 minutes at room temperature.
4. Centrifuge at 200 g during 5 minutes and remove the supernatant so as to eliminate the excess polyelectrolyte.
5. Carry out 2 washes with the same pellet to HBSS volume ratio of 1:10, using centrifugation at 200 g during 5 minutes.
6. Resuspend the obtained pellet at a pellet to polyanion ratio of 1:10 (hyaluronic acid to Chondroitin Sulfate ratio of 1:1.1 mg/mL). Incubate during 10 minutes at room temperature. Hyaluronic acid, potassium salt, human umbilical cord (H7980-13 US Biologicals). Chondroitin sulfate, bovine trachea, Mw 50000 (230699 Calbiochem).
7. Centrifuge at 200 g during 5 minutes and remove excess polyelectrolyte.
8. Carry out 2 washes considering a pellet to HBSS ratio of 1:10 with centrifugation at 200 g during 5 minutes.
9. Execute the same aforementioned process for each layer deposition, alternating the polyelectrolytes to maintain the opposing ionic interactions in layer-by-layer, according to the desired number of layers.

**Table 1 describes examples of the various formulations obtained by means of the present Invention.**

| **TABLE 1 (Examples)** | **Formation 1** | **Formation 2** | **Formation 3** | **Formation 4** | **Formation 5** | **Formation 6** | **Formation 7** | **Formation 8** |
|---|---|---|---|---|---|---|---|---|
| **CELLS (N° cells/mL)** | 1.0 X 10⁶ | 1.5 X 10⁶ | 0.5 X 10⁶ | 2.0 X 10⁶ | - | - | - | 2.0 X 10⁶ |
| **NANOENCAPSULATED CELLS (N° cells/mL)** | - | - | - | - | 1.0 X 10⁶ | 1.5 X 10⁶ | 0.5 X 10⁶ | - |

| **EXCIPIENT** | | | | | | | | |
|---|---|---|---|---|---|---|---|---|
| HEPES (mM) | 25 | - | 15 | 20 | 10 | - | 15 | - |
| Coloring agent FDC Blue N°1 (µM) | 100 | 50 | 25 | - | - | - | - | 50 |
| Coloring agent FDC Blue N°2 (µM) | - | - | - | 100 | 50 | 100 | 50 | - |
| Heparin (UI/mL) | - | - | - | 25 | 50 | 10 | 25 | - |
| Dalteparin (UI/mL) | - | 4 | 4 | - | - | - | - | - |
| Platelet lysate (%) | - | 20 | 10 | 20 | 10 | 5 | 20 | - |
| N-acetyl cysteine (mM) | - | 4 | 2 | 4 | - | 4 | 2 | 2 |

| **HEALING AGENTS** | | | | | | | | |
|---|---|---|---|---|---|---|---|---|
| Hyaluronic acid (mg/mL) | 0.05 | 0.2 | 0.2 | 0.2 | 0.2 | - | - | - |
| Allantoin (%p/v) | - | 0.4 | - | 0.2 | - | 0.2 | - | - |
| Chitosan (%p/v) | - | - | 0.15 | 0.15 | - | - | 0.15 | - |

| **ANTIBIOTIC SUBSTANCE** | | | | | | | | |
|---|---|---|---|---|---|---|---|---|
| Penicillin (UI/mL) | - | 100 | 100 | - | 100 | 100 | 100 | 100 |
| Streptomycin (µg/mL) | - | 100 | 100 | - | 100 | 100 | 100 | 100 |
| Gentamicin (µg/mL) | 20 | - | - | 20 | - | - | - | - |
| Ciprofloxacin (mg/mL) | - | 1 | - | - | - | 1 | - | 1 |
| Lactoferrin (µg/mL) | - | - | 50 | 100 | 50 | - | - | - |

| **CARRIER (q.s 20mL)** | | | | | | | | |
|---|---|---|---|---|---|---|---|---|
| Saline solution q.s. | - | - | + | - | - | - | + | - |
| HAM's medium q.s. | - | - | - | + | - | - | - | - |
| Ringer's serum q.s. | - | - | - | - | + | - | - | - |
| Ringer's lactate serum q.s. | + | - | - | - | - | - | - | - |
| Phosphate buffered saline (PBS) q.s. | - | + | - | - | - | + | - | - |
| AB serum q.s. | - | - | - | - | - | - | - | + |

### Example 3

### Determining Cell Viability

To determine the viability of cells and nanoencapsulated cells, and the viability thereof after the fine mist spray process, utilize the LIVE/DEAD kit, which uses tracer probes for calcein-AM and ethidium homodimer, and follow the Manufacturer's instructions (Figure 3).

In addition, the determination of cell viability after the atomization process - both for nanoencapsulated and non nanoencapsulated cells - is done with the help of a flow cytometry by incorporating propidium iodide. To this end, two cell aliquots are stained (one before and the other after atomizing them), incubating them with a propidium iodide solution (10 µg/mL) during 5-10 minutes at room temperature. Lastly, the cells are analyzed by flow cytometry to count the live cells and the dead cells.

The proliferation is determined with a MTT-based kit, following the Manufacturer's instructions.

The mesenchymal stem cell differentiation capacity is determined by following established protocols previously described in the state of the art (17, 18, and 19).

In short, for the differentiation into adipocyte, the stem cells are incubated in the presence of dexamethasone (1 µM), isobutyl-methyl xanthine (IBMX, 0.5 mM), indomethacin (100 µM) and insulin (10 µg/mL). After 21 days, the cells are stained with Oil Red O.

For the differentiation into osteoblast, the stem cells are incubated in the presence of dexamethasone (1 µM), β-glycerophosphate (10 mM) and ascorbate 2-phosphate (50 µg/mL). After 28 days, the cells are stained with Alizarin Red S.

### Analysis of Results and Discussion

In the present Invention it has been demonstrated that the fine mist spray process does not alter the cell viability of mesenchymal stem cells derived from human adipose tissue (Figure 2).

This surprising result opens the possibility of multiple uses of cell atomization (fine mist spraying) and the present Invention provides different pharmaceutical formulations for use in the healing of wounds and burns in general.

The treatment of wounds to date has been based on the use of dressings. Among the disadvantages of advanced healing dressings, one must consider that none of them by itself possesses all the characteristics or requirements that a dressing should have for treating all wound types, that is, necrotic, sloughing, granulating and epithelializing wounds.

This means that there is no single effective dressing or treatment for all wounds like the one that the present Invention proposes, which can be applied to diverse wounds originated by multiple causes, with the resulting advantage of the preparation of pharmaceutical formulations that facilitates the application on any wound type.

Furthermore, although advanced healing of wounds is effective in a large number of cases, there still are about 50% of patients whose wounds do not heal with conventional therapies, a situation that is even worse when a wound is over a year old.

The present Invention represents a single product for the treatment of wounds, which is effective for any wound type and wound age.

The fine mist spray application method described in the present Invention allows a faster healing of wounds, thereby reducing the hospitalization and nursing care times that a patient with different wound types requires.

Therefore, the product developed in the course of the present Invention is indicated for the chronic wounds that present insufficient pathological healing, according to those described in Table 2.

**Table 2. Causes of Insufficient Pathological Healing**

| |
|---|
| Pressure ulcers (bedsores) |
| Vascular insufficiency (arterial and venous) |
| Metabolic (diabetes, gout) |
| Infectious (bacteria, fungi, parasites) |
| Inflammatory (gangrenous pyoderma, vasculitis) |
| Hematological (polycythemia, fickle cells, hypercoagulability) |
| Malignancy (Marjolin's, primary and secondary tumors, Kaposi) |
| Miscellaneous (burns, radiation, frostbite) |

The present Invention also demonstrates that the nanoencapsulation of stem cells provides advantages with regard of non nanoencapsulated cells, which constitutes one more advantage to those previously mentioned. This can be seen in Figure 3: the nanoencapsulation process (Bar C) does not affect cell viability with respect to non nanoencapsulated cells (Bar A). The nanoencapsulated cells resist the fine mist spraying process better, a difference that attains statistical significance (p<0.05). * and # present a value of p<0.05 with regard to their pre-atomization control.

### REFERENCES:

1. Ikada Y. Challenges in tissue engineering. J R Soc Interface 2006; 3: 589-601.
2. Uludag H, De Vos P, Tresco PA. Technology of mammalian cell encapsulation. Adv Drug Deliv Rev 2000; 42: 29-64. 3.
3. Wilson JT, Chaikof EL. Challenges and emerging technologies in the immunoisolation of cells and tissues. Adv Drug Deliv Rev 2008; 60: 124-45.
4. Schaffler A, Buchler C. Concise review: adipose tissue-derived stromal cells--basic and clinical implications for novel cell-based therapies. Stem Cells 2007; 25: 818-27.
5. Puissant B, Barreau C, Bourin P, et al. Immunomodulatory effect of human adipose tissue-derived adult stem cells: comparison with bone marrow mesenchymal stem cells. Br J Haematol 2005; 129: 118-29.
6. Le Blanc K, Ringden O. Immunomodulation by mesenchymal stem cells and clinical experience. J Intern Med 2007; 262: 509-25.
7. Young HE, Duplaa C, Katz R, et al. Adult-derived stem cells and their potential for use in tissue repair and molecular medicine. J Cell Mol Med 2005; 9: 753-69.
8. Mizuno H. Adipose-derived stem cells for tissue repair and regeneration: ten years of research and a literature review. J Nippon Med Sch 2009; 76: 56-66.
9. Krol S, Diaspro A, Magrassi R, et al. Nanocapsules: coating for living cells. IEEE Trans Nanobioscience 2004; 3: 32-8.
10. Hillberg AL, Tabrizian M. Biorecognition through layer-by-layer polyelectrolyte assembly: in-situ hybridization on living cells. Biomacromolecules 2006; 7: 2742-50.
11. Krol S, Cavalleri O, Ramoino P, Gliozzi A, Diaspro A. Encapsulated yeast cells inside Paramecium primaurelia: a model system for protection capability of polyelectrolyte shells. J Microsc 2003; 212: 239-43.
12.Veerabadran NG, Goli PL, Stewart-Clark SS, Lvov YM, Mills DK. Nanoencapsulation of stem cells within polyelectrolyte multilayer shells. Macromol Biosci 2007; 7: 877-82.
13.Sorrel M, Caplan A.: "Topical delivery of mesenchymal stem cell and their function in wounds". Stem Cell Research & Thetapy 2010, 1:30
14. Falanga V, Iwamoto S, Chartier M, Yufit T, Butmarc J, Kouttab N, Shrayer D, Carson P: "Autologous bone marrow-derived cultured mesenchymal stem cells delivered in a fibrin spray accelerate healing in murine and human cutaneous wounds". Tissue Eng. 2007 Jun; 13(6): 1299-312.ANGA
15.Cha J & Falanga V. Stem cells in cutaneous wound healing. Clin Dermatol. 2007; 25: 73-78
16.Vermeulen H, Ubbink D, Goossens A, de Vos R, Legemate D: Apósitos y agentes tópicos para heridas quirúrgicas que cicatrizan por segunda intención (Revisión Cochrane traducida). En: La Biblioteca Cochrane Plus, 2006 Número 1. Oxford: Update Software Ltd. Disponible en: http://www.update-software.com. (Traducida de The Cochrane Library, 2006 Issue 1. Chichester, UK: John Wiley & Sons, Ltd.).
17. Bunnell BA, Flaat M, Gagliardi C, Patel B, Ripoll C. Adipose-derived stem cells: Isolation, expansion and differentiation. Methods 2008; 45: 115-120.
18.Schäffler A and Büchler C. Concise review: Adipose tissue-derived stromal cells - Basic and clinical implications for novel cell-based therapies. Stem Cells 2007; 25: 818-827.
19. Gomillion CT and Burg KJL. Stem cells and adipose tissue engineering. Biomaterials 2006; 27: 6052-6063

## Claims

1. Topical fine mist spray application system **CHARACTERIZED IN THAT** it comprises a container or vessel with an atomizer and a pharmaceutical formulation wherein the active ingredient consists in live cells suspended in an acceptable pharmaceutical carrier.

2. Topical fine mist spray application system according to Claim 1, **CHARACTERIZED IN THAT** the live cells of the active ingredient correspond to mesenchymal stem cells.

3. Topical fine mist spray application system according to Claim 1, **CHARACTERIZED IN THAT** the live cells of the active ingredient can be nanoencapsulated or non nanoencapsulated.

4. Topical fine mist spray application system according to Claim 2, **CHARACTERIZED IN THAT** the mesenchymal stem cells can be nanoencapsulated or non nanoencapsulated.

5. Topical fine mist spray application system according to Claim 2, **CHARACTERIZED IN THAT** the mesenchymal stem cells present the capacity of differentiating themselves into adipose cells, osteoblasts and chondrocytes.

6. Topical fine mist spray application system according to Claim 2, **CHARACTERIZED IN THAT** the mesenchymal stem cells maintain cell viability and integrity during a set period of time.

7. Topical fine mist spray application system according to Claim 6, **CHARACTERIZED IN THAT** the cell viability and integrity period comprises between 1 hour and 72 hours.

8. Topical fine mist spray application system according to Claim 1, **CHARACTERIZED IN THAT** the pharmaceutically acceptable carrier is selected from among the group formed by saline solution, phosphate buffered saline (PBS), Ringer's serum, Ringer's lactate serum, autologous serum, and AB donor serum.

9. Topical fine mist spray application system according to Claim 8, **CHARACTERIZED IN THAT** the pharmaceutically acceptable carrier also comprises agents that promote wound healing and agents that ensure sterility of the pharmaceutical formulation.

10. Topical fine mist spray application system according to Claim 9, **CHARACTERIZED IN THAT** the agents that promote wound healing are selected from a group that includes chitosan, hyaluronic acid, allantoin, type 1 collagen, a decoction or extract (tannins) of matico *(Piper angustifolium),* a tincture of hydrocotyle or *Centella asiatica* (could be alcoholic), decoctions of calendula *(Calendula officinalis)* and Aloe Vera extracts.

11. Topical fine mist spray application system according to Claim 9, **CHARACTERIZED IN THAT** the agents that ensure sterility are selected from amongst penicillin, streptomycin, gentamicin, quinolones, silver sulfadiazine, chlorhexidine, quaternary ammonium derivatives and lactoferrin.

12. Topical fine mist spray application system according to Claim 8, **CHARACTERIZED IN THAT** the pharmaceutically acceptable carrier further comprises 4-(2-hydroxyetyl)-1-piperazine-ethanosulfonic acid (HEPES) buffer, bicarbonate buffer, FDC coloring agents (for example, Blue N°1 and Blue N°2), heparins, platelet lysates, and N-acetyl cysteine (NAC).

13. Topical fine mist spray application system according to Claim 1, **CHARACTERIZED IN THAT** said system comprises a container or vessel made of sterile glass having a threaded neck so as to allow adjusting a fine mist spray system.

14. Topical fine mist spray application system according to Claim 13, **CHARACTERIZED IN THAT** the volume of the vessel ranges between 5 mL and 20 mL.

15. Topical fine mist spray application system according to Claim 1, **CHARACTERIZED IN THAT** the cell concentration in the pharmaceutical formulation ranges between 5 x 10⁵ and 4 x 10⁶ cells per mL.

16. Pharmaceutical formulation to be applied in spray form **CHARACTERIZED IN THAT** it comprises an active ingredient consisting in live cells suspended in a pharmaceutically acceptable carrier.

17. Pharmaceutical formulation according to Claim 16, **CHARACTERIZED IN THAT** the live cells of the active ingredient correspond to mesenchymal stem cells.

18. Pharmaceutical formulation according to Claim 16, **CHARACTERIZED IN THAT** the live cells of the active ingredient can be nanoencapsulated or non nanoencapsulated.

19. Pharmaceutical formulation according to Claim 17, **CHARACTERIZED IN THAT** the mesenchymal stem cells can be nanoencapsulated or non nanoencapsulated.

20. Pharmaceutical formulation according to Claim 17, **CHARACTERIZED IN THAT** the mesenchymal stem cells present the capacity to differentiate themselves into adipose cells, osteoblasts and chondrocytes.

21. Pharmaceutical formulation according to Claim 17, **CHARACTERIZED IN THAT** the mesenchymal stem cells maintain cell viability and integrity during a set period of time.

22. Pharmaceutical formulation according to Claim 21, **CHARACTERIZED IN THAT** the cell viability and integrity period comprises between 1 hour and 72 hours.

23. Pharmaceutical formulation according to Claim 16, **CHARACTERIZED IN THAT** the pharmaceutically acceptable carrier is selected from the group formed by saline solution, phosphate buffered saline (PBS), Ringer's serum, Ringer's lactate serum, autologous serum, and AB donor serum.

24. Pharmaceutical formulation according to Claim 23, **CHARACTERIZED IN THAT** the pharmaceutically acceptable carrier also comprises agents that promote wound healing and agents that ensure sterility of the pharmaceutical formulation.

25. Pharmaceutical formulation according to Claim 24, **CHARACTERIZED IN THAT** the agents that promote wound healing are selected from amongst chitosan, hyaluronic acid, allantoin, type 1 collagen, a decoction or extract (tannins) of matico *(Piper angustifolium),* a tincture of hydrocotyle or *Centella asiatica* (could be alcoholic), decoctions of calendula *(Calendula officinalis)* and Aloe Vera extracts.

26. Pharmaceutical formulation according to Claim 24, **CHARACTERIZED IN THAT** the agents that ensure sterility are selected from amongst penicillin, streptomycin, gentamicin, quinolones, silver sulfadiazine, chlorhexidine, quaternary ammonium derivatives and lactoferrin.

27. Pharmaceutical formulation according to Claim 23, **CHARACTERIZED IN THAT** the pharmaceutically acceptable carrier also comprises 4-(2-hydroxyetyl)-1-piperazine-ethanosulfonic acid (HEPES) buffer, bicarbonate buffer, FDC coloring agents (for example, Blue N°1 and Blue N°2), heparins, platelet lysates, and N-acetyl cysteine (NAC).

28. Pharmaceutical formulation according to Claim 16, **CHARACTERIZED IN THAT** the cell concentration of the pharmaceutical formulation ranges between 5 x 10⁵ and 4 x 10⁶ cells per mL.

29. Use of the pharmaceutical formulation according to Claim 16, **CHARACTERIZED IN THAT** it is useful for preparing a sprayable pharmaceutical composition intended for topical application in humans and animals.

30. Use of the pharmaceutical formulation according to Claim 29, **CHARACTERIZED IN THAT** it is useful for preparing a sprayable pharmaceutical composition intended for treating wounds.

31. Use of the pharmaceutical formulation according to Claim 30, **CHARACTERIZED IN THAT** the wounds are chronic wounds.

32. Use of the pharmaceutical formulation according to Claim 31, **CHARACTERIZED IN THAT** the chronic wounds are the result of pressure ulcers (bedsores), vascular insufficiency (arterial and venous), metabolic disorders (diabetes, gout), infections (bacteria, fungi, parasites), inflammations (gangrenous pyoderma, vasculitis), hematological disorders (polycythemia, fickle cells, hypercoagulability), cellular malignancy (Marjolin's, primary and secondary tumors, Kaposi), and other wounds caused by burns, radiation, frostbite.
